# EUROPEAN PATENT APPLICATION

(11) **EP 2 070 522 A1**
(43) Date of publication of application: **17.06.2009**
(21) Application number: 07291487.2
(22) Date of filing: 11.12.2007
(51) Int. Cl.: A61K 31/00

(54) **Compounds for preventing and treating plasmodium infections.**

(71) Applicant: UNIVERSITE PIERRE ET MARIE CURIE (PARIS VI), 75005 Paris (FR)
(72) Inventor: Mazier, Dominique, 75010 Paris (FR); Mahmoudi, Nassira, 77340 Pontault-Combault (FR); Farhati, Khémaïs, 93130 Noisy le Sec (FR); García-Domenech, Ramon, 46100 Valencia (ES); Gàlvez, Jorge, 46100 Valencia (ES); Derouin, Francis, 78100 Saint Germain en Laye (FR); Danis, Martin, 75006 Paris (FR)
(74) Representative: Colombet, Alain André

(57) **Abstract**

The present invention relates to the use of at least one compound of the following formula (I): 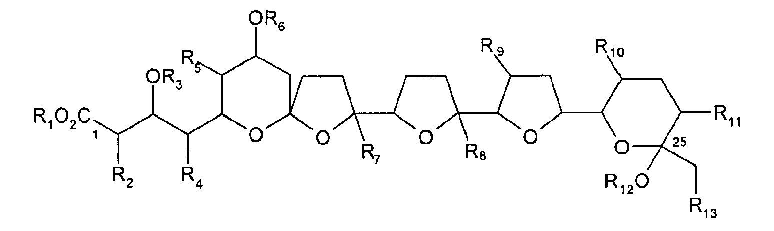 for the manufacture of a medicament intended for preventing or treating infections by a *Plasmodium* parasite in an individual, by inhibiting the pre-erythrocytic development stage of said *Plasmodium* parasite.

SEQUENCE LISTING

<110> Universite Pierre et Marie Curie

<120> Compounds and methods for preventing and treating Plasmodium
      infections

<130> BET 07P1457

<160> 6

<170> PatentIn version 3.4

<210> 1
<211> 25
<212> DNA
<213> Artificial sequence

<220>
<223> Primer

<400> 1
ttagattttc tggagacaaa caact                                          25


<210> 2
<211> 22
<212> DNA
<213> Artificial sequence

<220>
<223> Primer

<400> 2
tcccttaact ttcgttcttg at                                             22


<210> 3
<211> 28
<212> DNA
<213> Artificial sequence

<220>
<223> Primer

<400> 3
cgaaagcatt tgcctaaaat acttccat                                       28


<210> 4
<211> 21
<212> DNA
<213> Artificial sequence

<220>
<223> Primer

<400> 4
acggccaggt catcactatt g                                              21

<210>  5
<211>  21
<212>  DNA
<213>  Artificial sequence

<220>
<223>  Primer

<400>  5
caagaaggaa ggctggaaaa g                                                21


<210>  6
<211>  21
<212>  DNA
<213>  Artificial sequence

<220>
<223>  Primer

<400>  6
caacgagcgg ttccgatgcc c                                                21

## Description

### Field of the invention

The present invention relates to compounds and methods useful for preventing and treating *Plasmodium* infections.

### Background of the invention

The global spread of multidrug-resistant malaria parasites has led to an urgent need for new chemotherapeutic agents targeting multiplication of the parasite in erythrocytes (the process responsible for the pathology). This is an obvious necessity, and most resources available to malaria research are devoted to this aim, particularly for new artemisinin based combined treatments (ACT). However, before multiplying in the blood, the parasite undergoes multiplication in the liver.

Indeed the malaria parasite exhibits a complex life cycle involving an insect vector, mosquito, and a vertebrate host. Four main *Plasmodium* species infect humans: *P*. *falciparum*, *P*. *vivax*, *P*. *ovale* and *P*. *malariae*. All four species exhibit a similar life cycle with only minor variations.

The infection is initiated when sporozoites are injected with the saliva of a feeding mosquito. Sporozoites are carried to the liver and invade hepatocytes. The intracellular parasite undergoes an asexual replication known as exoerythrocytic schizogony within the hepatocyte. This stage is also known as the liver (hepatocytic) stage or the pre-erytnrocytic (PE) development stage. Exoerythrocytic schizogony culminates in the production of merozoites which are released into the bloodstream. A proportion of the liver stage parasites from *P*. *vivax* and *P*. *ovale* go through a dormant period instead of immediately undergoing asexual replication. These hypnozoites will reactivate several weeks to months (or years) after the primary infection and are responsible for relapses.

Merozoites invade erythrocytes, thus beginning the erythrocytic development stage, or blood stage, and undergo a trophic period in which the parasite enlarges. The early trophozoite is often referred to as "ring form" because of its morphology. Trophozoite enlargement is accompanied by an active metabolism including the ingestion of host cytoplasm and the proteolysis of hemoglobin into amino acids. The end of the trophic period is manifested by multiple rounds of nuclear division without cytokinesis resulting is a schizont. Merozoites bud from the mature schizont, also called a segmenter, and the merozoites are released following rupture of the infected erythrocyte. Invasion of erythrocytes reinitiates another round of the blood stage replicative cycle.

The blood stage is responsible for the pathology associated with malaria.

As an alternative to the asexual replicative cycle, the parasite can differentiate into sexual forms known as macro- or microgametocytes. The gametocytes are large parasites which fill up the erythrocyte, but only contain one nucleus. Ingestion of gametocytes by the mosquito vector induces gametogenesis and escape from the host erythrocyte. Microgametes, formed by a process known as exflagellation, are flagellated forms which will fertilize the macrogamete leading to a zygote.

The zygote develops into a motile ookinete which penetrates the gut epithelial cells and develops into an oocyst. The oocyst undergoes multiple rounds of asexual replication resulting in the production of sporozoites. Rupture of the mature oocyst releases the sporozoites into the hemocoel (*i*.*e*. the body cavity) of the mosquito. The sporozoites migrate to and invade the salivary glands, thus completing the life cycle.

Accordingly, full inhibition of the pre-erythroctic (PE) development stage of the infection provides true causal prophylaxis whereby the blood stage infection and its associated clinical manifestations would be totally prevented. Besides, for *P*. *vivax and P*. *ovale*, drugs active against hypnozoites would provide a radical cure of the infection.

However, in drug discovery programs, routine screening for anti-PE stage activity is seldom carried out, partly because these stages are clinically silent, but mainly because access to PE parasites is costly and restricted to a few laboratories. Consequently, there are a limited number of drugs for human use effective against the *Plasmodium* liver stage parasite.

The deployment of primaquine, the only drug specifically developed to inhibit the liver infection, has been curtailed by the associated toxicity, poor compliance, and increased risk of haemolysis when administered to persons with glucose-6-phosphate dehydrogenase deficiency. This latter problem will also affect the two related synthetic 8- aminoquinolines, bulaquine (Valecha et al, 2001) and tafenoquine (Walsh et al, 2004), presently undergoing clinical trials.

Antifolates and atovaquone, primarily used in combination to treat the blood stage infection, were also shown to be active against the infected hepatocyte. However, the high prevalence of resistant parasites to the former, and the ease with which resistance arises to the latter, limit the prophylactic usefulness of these drugs. Moreover, these compounds could not be shown to be active against hypnozoites.

Quinine, chloroquine, mefloquine, and artemisinin-based compounds, have little or no efficacy against the hepatic parasite.

Other compounds may have an anti-pre-erythrocytic stage activity in humans (Neerja et al, 2004; Singh et al, 1998; Lindenthal et al, 2005; Puri et al, 1990, Guan et al, 2005; Zhang et al, 2005; Carraz et al, 2006), however none of them has reached clinical testing for the moment.

Accordingly, there is a continued need for new compounds liable to target the pre-erythrocytic development stage of *Plasmodium* parasites.

Monensin, is a natural antibiotic isolated from *Streptomyces cinnamonensis*. Two main forms of Monensin have been described in *Streptomyces cinnamonensis*, Monensin A and Monensin B,.of the following formulae: 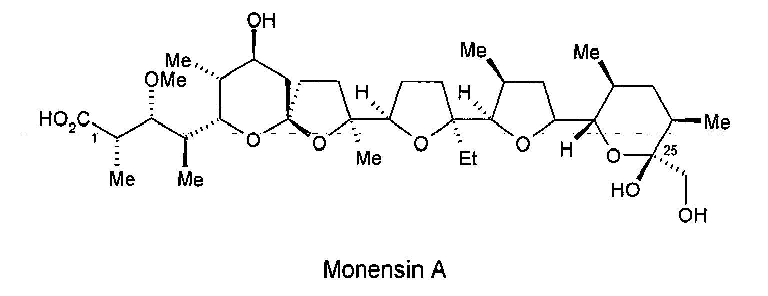 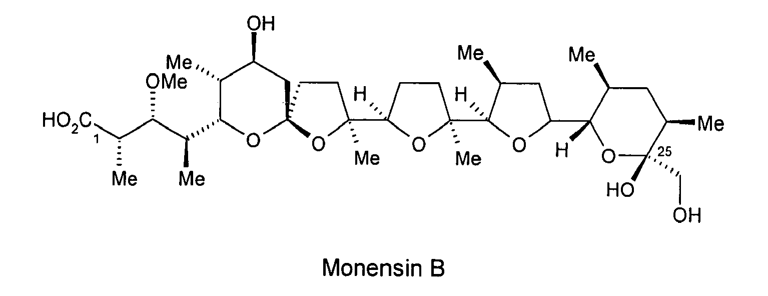 Monensin belongs to the class of the polyether ionophore antibiotics and forms pseudomacrocyclic complexes with monovalent and divalent cations which it transports across cellular membranes. Monensin A and a methyl ether derivative of Monensin A were reported to treat mice infected by *P*. *chabaudi* and *P*. *vinckei petteri* parasitized erythrocytes (i.e. by targeting only the blood stage) (Gumila et al, 1997).

### Description of the invention

The present invention arises from the unexpected finding, by the inventors, that Monensin A prevents the development of malaria in mice challenged with *P*. *yoelii* sporozoites.

Thus, the present invention relates to the use of at least one compound of the following formula (I): 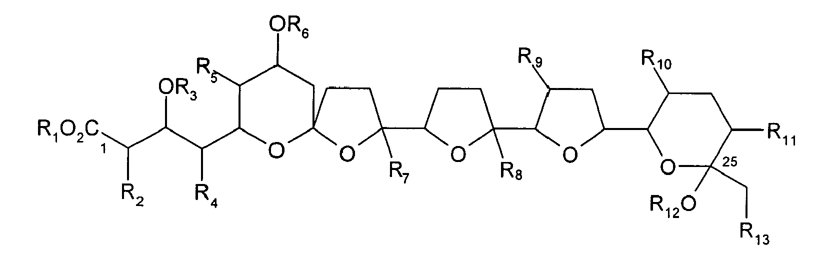 wherein:
- independently from each other, R₁ to R₁₂ represent H or an alkyl group having from 1 to 5 carbon atoms;
- R₁₃ represents a group selected from the list constituted of -OR, -NHR, - OCONHR, -OCOR₁₄, wherein R₁₄ represents a group selected from the list constituted of an alkyl having from 1 to 5 carbon atoms, an aryl having from 5 to 8 carbon atoms, an alkylaryl having from 5 to 13 carbon atoms, an arylalkyl having from 5 to 13 carbon atoms, a haloaryl having from 5 to 8 carbon atoms, an alkylhaloaryl having from 5 to 13 carbon atoms, and a haloarylalkyl having from 5 to 13 carbon atoms; or a pharmacologically acceptable salt thereof, for the manufacture of a medicament intended for preventing or treating infections by a *Plasmodium* parasite in an individual, by inhibiting the pre-erythrocytic development stage of said *Plasmodium* parasite.

The present invention also relates to a method for preventing or treating infections by a *Plasmodium* parasite in an individual, by inhibiting the pre-erythrocytic development stage of said *Plasmodium* parasite, wherein a prophylactically or therapeutically effective quantity of a compound of formula (I) as defined above is administered to said individual.

The present invention also relates to products containing:
- at least one compound of formula (I) as defined above, and
- at least one compound having anti-malarial activity, as a combined preparation for simultaneous, separate or sequential use for the prevention or the treatment of infections by a *Plasmodium* parasite in an individual, by inhibiting the pre-erythrocytic development stage of said *Plasmodium* parasite.

As intended herein, the compounds of formula (I) as defined above specifically inhibit the development of *Plasmodium* sporozoites into forms capable of infecting erythrocytes, *i*.*e*. the compounds of formula (I) as defined above target and impairs the liver or hepatic stage of the life cycle of *Plasmodium* parasites. Without being bound to any particular theory, it is believed that the compound of formula (I) as defined above directly impairs the ability of *Plasmodium* sporozoites to invade and develop in hepatocytes. As the compounds of formula (I) as defined above target the first stage of *Plasmodium* infection, these compounds are particularly useful for preventing the infection by the *Plasmodium* parasite and for preventing the symptoms associated to the *Plasmodium* parasite infection, such as erythrocyte destruction. As such, compounds of formula (I) as defined above are preferably intended for individual who have not been infected by a *Plasmodium* parasite or for asymptomatic individuals which have been exposed or have been at risk of having been exposed to an infection by *Plasmodium* sporozoites. Thus, where the individual is a human, it is preferably a pregnant woman or a child.

As intended herein, the individual is preferably a mammal, more preferably a human.

As intended herein, the *Plasmodium* parasite relates to any parasite of the *Plasmodium* genus. However, it is preferably one that is associated with malaria in mammals, in particular the human form of malaria. As such, the *Plasmodium* parasite is preferably selected from the list constituted of *P*. *falciparum*, *P*. *vivax*, *P*. *ovale* and *P*. *malariae*.

The compounds of formula (I) can readily be synthesized or obtained by one of skill in the art. By way of example, besides Monesin A and B which can be isolated from *Streptomyces cinnamonensis*, US 4 294 925 describes the obtaining compounds of formula (I) from particular strains of the *Streptomyces* genus. The obtention of a plurality of compounds of formula (I) are also described in Gaboyard et al. (1990) Agric. Biol. Chem. 54:1149-1155, Tanaka et al. (2001) Chem. Pharm. Bull. 49:711-715 and Smith & Still (1988) J. Am. Chem. Soc. 110:7917-7919.

Generally speaking, the scope of the invention should be construed as extending to any compound liable to be derived from Monensin by chemical modification and presenting the effects of Monensin on *Plasmodium* sprozoites or on *Plasmodium* sporozoites infection, wherein the effects are in particular assayed as described in the following examples.

Preferably, however, the compound of formula (I) as defined above is of the following formula (II): 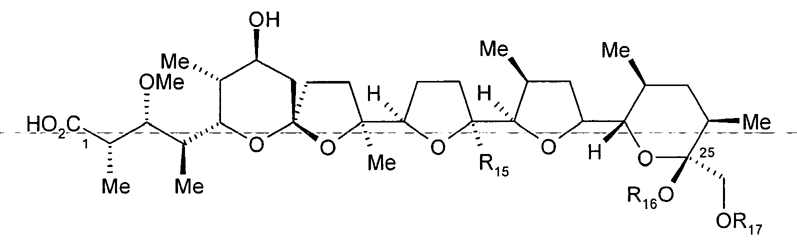 wherein:
R₁₅ represents methyl or ethyl;
R₁₆ represents H or methyl;
R₁₇ represents H or -CONHR₁₈, wherein R₁₈ represents a group selected from the list constituted of phenyl, p-chlorophenyl or p-bromophenyl.

More preferably, the compound of formula (I) is selected from the group constituted of: 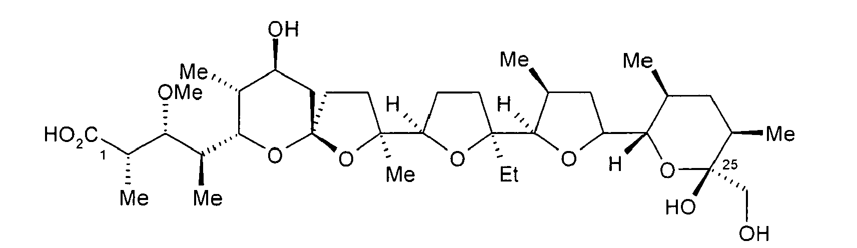 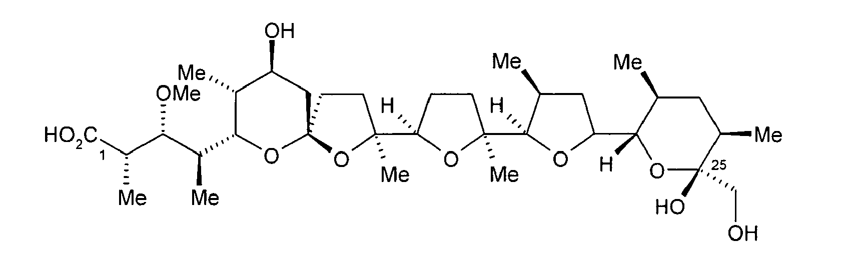 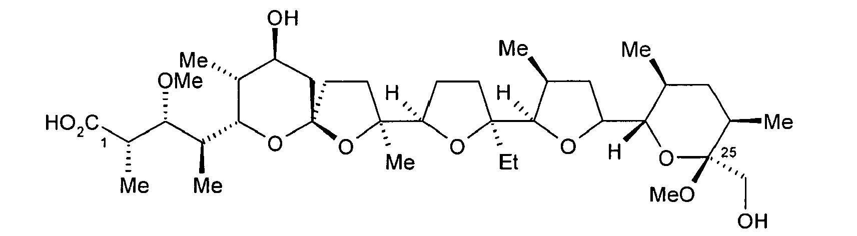 and 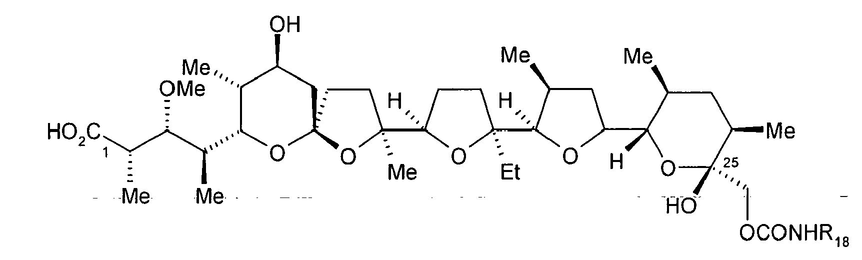 wherein R₁₈ is as previously defined.

Most preferably, the compound of formula (I) is Monensin A, of the following formula (III):

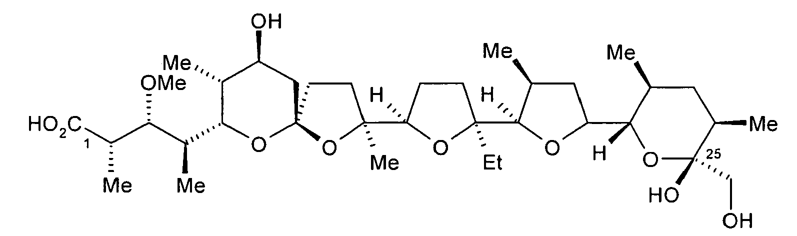

Preferably, the medicament is suitable for a dose regimen of from 1 to 15 mg/kg/day of the compound, more preferably of about 7 or 8 mg/kg/day.

Preferably, the medicament comprises a unit dose of from 10 mg to 2 g of the compound, more preferably of from 70 mg to 600 mg.

Preferably, the medicament is suitable for administration by the oral route.

Preferably also, the medicament further comprises at least one compound having anti-malarial activity.

Preferably, the compound having anti-malarial activity as defined above is selected from the group consisting of Atovaquone, Proguanil, Quinine, Quinidine, Quinine-doxycycline, Artemether, Artemotil, Artesunate, Arteether, Méfloquine, Amodiaquine, Dihydroartémisinine, Pipéraquine, Halofantrine, Atovaquone, Chloroquine, Dapsone, Doxycycline, a Cycline, Lumefanthrine, Proguanil, Pyrimethamine, Pyronaridine, Sulfadoxine, Diamidine, Ferroquine, Fluoroquinolone, Fosmidomycine, Tafenoquine, and Trioxaquine. More preferably, the compound having anti-malarial activity as defined above is Atovaquone or Proguanil; most preferably it is Proguanil.

### BRIEF DESCRIPTION OF THE FIGURES

### Figure 1

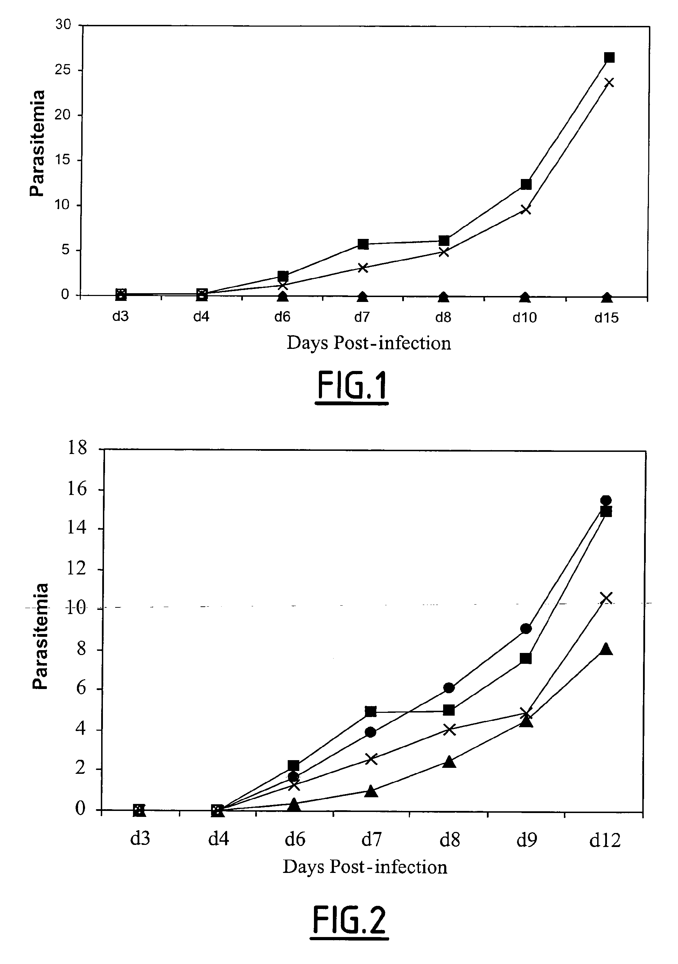

Figure 1 represents the parasitemia (percentage of infected red blood cells) (y-axis) of mice infected by *P*. *yoelii* sporozoites and treated by 25 mg/kg Monensin (MN) for one day before infection and 3 days after infection (diamonds), for 3 days after infection (triangles), or untreated (squares, crosses), as a function of time (x axis, days post infection).

### Figure 2

Figure 2 represents the parasitemia (percentage of infected red blood cells) (y-axis) of mice infected by *P*. *yoelii* sporozoites and treated by 35 mg/kg Nigericin (NG) for one day before infection and 3 days after infection (diamonds), for 3 days after infection (triangles), or untreated (squares, crosses), as a function of time (x-axis, days post infection).

### Figure 3

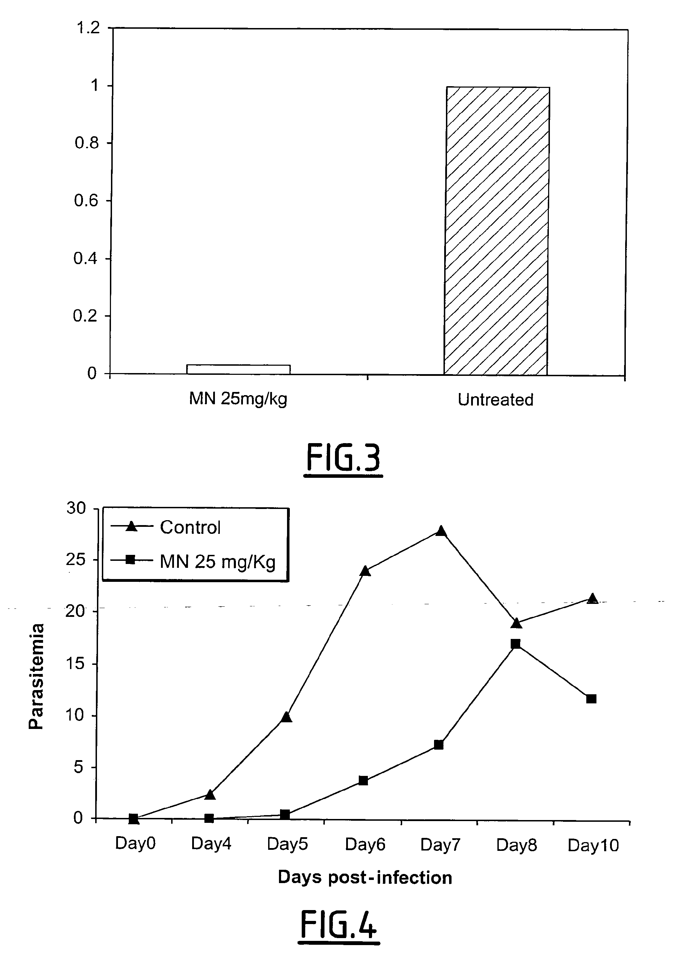

Figure 3 represents the ratio of the quantitative parasite burden determined by real time PCR in the liver of mice infected by *P*. *yoelii* sporozoites and treated by 25 mg/kg MN versus that of untreated mice.

### Figure 4

Figure 4 represents the parasitemia (percentage of infected red blood cells) (y-axis) of mice infected by *P*. *yoelii* blood stage and treated by 25 mg/kg Monensin (MN) for three days after infection (squares, crosses), or untreated (triangles), as a function of time (x-axis, days post infection).

### Figure 5

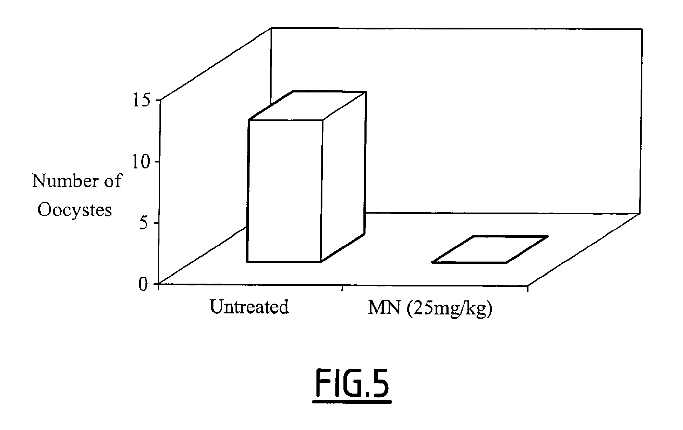

Figure 5 represents the number of oocystes in mosquitoes treated with 25mg/kg MN *versus* untreated mosquitoes.

### EXAMPLES

### Example 1: Drug treatment prevents development of erythrocytic infection after a challenge with Plasmodium yoelii sporozoites

### Material and method

All animal were kept and used in accordance with institutional guidelines and European regulations. Female 6-week-old Swiss mice (René Janvier, Le Genest-Saint-Isle, France) weighing 24-31 g were randomly allotted to all groups.

The drugs tested were administered intra-peritoneally (IP). Monensin (MN) (Monensin A sodium salt, ref. M5273, Sigma) was diluted in PBS-methanol 2% and Nigericin (NG) (Nigericin sodium salt, ref. N7143, Sigma), another polyether ionophore antibiotic, in PBS-DMSO 2%.

The drugs were administrated on days -1, 0, +1, +2 (+40 h), and the mice challenged on day 0 by intravenous injection of 5,000 *P*. *yoelii* sporozoites (265 BY strain).

Group 1 received 25 mg/kg/day of MN and group 2 received PBS-methanol 2%. These groups received the drug and the solvent from day -1. Group 3 received 25 mg/kg/day of MN and group 4 received PBS-methanol 2% from day 0 post-infection. Group 5 received 35 mg/kg/day of NG and group 6 received PBS-DMSO 2% from day -1. Group 7 and 8 received 35 mg/kg/day of NG and PBS-DMSO 2% respectively from day 0 post-infection.

All groups were challenged with 5,000 sporozoites at day 0. Parasites were enumerated by blinded microscopic examination of Giemsa-stained tail-blood smears collected at day 3 until day 9, day 12 and day 15 post-infection. A mouse was considered negative when no parasites could be observed in 20,000 red blood cells (50 fields under a 50× light microscope objective) on any day during the period of observation.

### Results

Upon challenge with 5,000 *P*. *yoelii* sporozoites, protection from erythrocytic infection was obtained in 100 % of the mice treated with MN daily for 3 days (treated) or 4 days (pre-treated) since no parasite could be observed observed in blood smears (**Figure 1**).

However, for the groups receiving NG daily for 3 days (treated) or 4 days (pretreated), no total protection could be evidenced. Indeed, only 51% and 71% of reduction of parasitemia with respect to controls was observed after 12 days **(****Figure 2****).**

### Example 2: Real-Time PCR quantification of the liver stage parasite burden

The anti-malarial activity of MN against the pre-erythrocytic development stage of *P*. *yoelii* was further evaluated *in vivo* by quantification of the number of parasites in the liver by Real-time PCR.

### Material and method

MN was administered IP on days 0 and day +1. Group 1 received 25 mg/kg of MN, whereas the control groups received the MN solvent (PBS-Methanol 2%). Mice were then challenged on day 0 by intra-venous injection with 2.5×10⁵ *P*. *yoelii* sporozoites and sacrificed 40 h post-infection.

A piece of liver (0.2 g) was harvested and total RNA extracted using the Micro to Midi Kit (Invitrogen, France) according to the manufacturer's instructions and treated with Dnase Turbo DNA free (Ambion, France). Five micrograms of total RNA was reverse transcribed by *Superscript* II (Invitrogen) and an equivalent of 100 ng RNA was used for each TaqMan^{®} PCR reactions on a MX4000 multiplex quantitative PCR system (Stratagene, France). *P*. *yoelii* Gene-specific primers (forward TTAGATTTTCTGGAGACAAACAACT (SEQ ID NO: 1), reverse TCCCTTAACTTTCGTTCTTGAT (SEQ ID NO: 2); Invitrogen) and probe (6-FAM-CGAAAGCATTTGCCTAAAATACTTCCAT-BHQ1 (SEQ ID NO: 3); MWG biotech) were selected from the *P*. *yoelii* 265By 18S rRNA sequence (GeneBank accession number: AF266261) using the Primer Express software (PE Applied Biosystems, France) and did not cross react with rodent DNA.

Mouse β -actin primers (forward ACGGCCAGGTCATCACTATTG (SEQ ID NO: 4), reverse CAAGAAGGAAGGCTGGAAAAG (SEQ ID NO: 5); Invitrogen) and probe (HEX-CAACGAGCGGTTCCGATGCCC-BHQ2 (SEQ ID NO: 6); MWG biotech) were used for normalisation. *P*. *yoelii* 18S rRNA and mouse β-actin PCR fragments were cloned into plasmids and used in a 10-fold dilution series to determine a standard curve.

Absolute transcript copy number for each gene was calculated based on the external standard curve. Quantitative parasite burden was expressed as the ratio between absolute copy numbers of *P*. *yoelii* 18S ribosomal cDNA and mouse β-actin cDNA to account for experimental differences. TaqMan^{®} test sample and plasmid standards were done in quadruplet.

### Results

96.8% inhibition on parasite development was obtained for MN treatment **(****Figure 3****).**

### Example 3: Monensin acts on P. yoelii sporozoïtes per se

The *in vitro* effect of MN on the migration and invasion properties of *P*. *yoelii* sporozoites was further studied.

### 1. Migration assay

### Material and method

*P*. *yoelii* sporozoites treated with three concentrations (5µM, 5nM and 5 picomolar) of MN were incubated with HepG2CD81 cells for 2h at 37°C in the continued presence of with 0.5 mg/ml FITC-dextran. After 2 h at 37°C, cells were washed and fixed with PBS-formaldehyde 1%, and FITC-positive cells were counted by flow cytometry.

### Results

All concentrations of MN showed an inhibitory effect on sporozoites migration (93% of inhibition) by comparison with untreated sporozoïtes.

### 2. Invasion assay

### Material and method

Two experiments were performed.

The first experiment consisted in pre-reatment of *P*. *yoelii* sporozoites with MN at 5 pM, 0.5 µM, or 5 µM, for 1 h at room temperature then washed and added to hepatocytes. Sporozoites controls were pre-treated with medium alone and incubated 1 h at room temperature in the same conditions. Parasites were then incubated with hepatocytes for 48h at 37°C in 5%CO₂, stained, and examined microscopically to determine the quantity of exo-erythrocytic forms (EEF) (Basco *et al*., 1999; Mahmoudi *et al*., 2003).

The second experiment consisted in infecting hepatocytes with sporozoites in the presence of MN at 50 pM, 1 µM, or 10 µM. After 3h of incubation, MN was eliminated and the cultures were maintained 45h at 37°C in 5% CO₂.

The cultures were stained and the quantity of exo-erythrocytic forms (EEF) was determined microscopically.

### Results

In the first experiment, complete inhibition of parasite development could be observed for 5µM and 0.5µM of MN, while a 96.26% inhibition was observed with a concentration of 5 pM.

In the second experiment, all concentrations tested of MN totally inhibited parasite development.

### Example 4: Monensin acts on P. yoelii maturation

The *in vitro* effect of MN on the development of P. *yoelii* sporozoites was also studied

### Material and method

The experiment consisted in infecting hepatocytes cultures with *P*. *yoelii* sporozoites for 3h, washing the cultures and then treating them with MN 50 pM, 1 µM, or 10 µM, for 45h at 37°C.

### Results

All concentrations of MN completely inhibited parasite development.

### Example 5: Prevention of erythrocytic infection is due to an effect at the hepatic level

To confirm that the anti-malarial activity observed *in vivo* was specifically directed against the pre-erythrocytic development stage of the *Plasmodium* parasite, the inventors have tested the inhibitory effect of Monensin against P. *yoelii* blood stage.

### Material and method

Two groups of mice were treated at day-3, day-2 and day -1 before infection with MN (25 mg/kg) or solvent (PBS-Methanol-2%). At day 0 the mice were inoculated IP with 4x10⁵ *P*. *yoelii*-infected red blood cells. From day 3 post-infection, an enumeration of parasites in blood smears was conducted until day 10 post-infection.

### Results

Increasing parasitemia, although delayed by comparison with the control group, could be evidenced in mice treated with MN **(****Figure 4****),** which indicates that the absence of parasitemia evidenced in **Example 1** is essentially linked to an effect on the pre-erythrocytic develoment stage of the *Plasmodium* parasite.

Interestingly, in the mice treated with MN, no male gametocytes were observed by comparison with the untreated group.

### Example 6: Monensin prevents transmission to mosquito

### Material and method

The mice of the MN treated group and the control group of **Example 3** were anesthetized and placed for 2 hours over a cage with 100 female laboratory-bred *Anopheles stephensi*. Oocysts were enumerated in 30 mosquito midguts dissected 8 days later.

### Results

No oocyst could be observed for the MN treated group by comparison with the untreated group **(****Figure 5****).**

### REFERENCES

Carraz M., Jossang A., Franetich J. F., Siau A., Ciceron L., Hannoun L., Sauerwein R., Frappier F., Rasoanaivo P., Snounou G. and Mazier D. (2006). A plant-derived morphinan as a novel lead compound active against malaria liver stages. PLoS Medecine 3(12): e513

Gego A., Silvie O., Franetich J. F., Farhati K., Hannoun L., Luty A. J., Sauerwein R. W., Boucheix C., Rubinstein E. and Mazier D. (2006). New approach for high-throughput screening of drug activity on Plasmodium liver stages. Antimicrob Agents Chemother 50(4): 1586-9.

Gumila C., Ancelin M. L., Delort A. M., Jeminet G., Vial H. J. (1997) Characterization of the potent in vitro and in vivo antimalarial activities of ionophore compounds. Antimicrob Agents Chemother. 41 (3): 523-9.

Guan J, Zhang Q, Montip G, Karle JM, Ditusa CA, et al. (2005) Structure identification and prophylactic antimalarial efficacy of 2-guanidinoimidazolidinedione derivatives. Bioorg Med Chem 13: 699-704.

Lindenthal C, Weich N, Chia YS, Heussler V, Klinkert MQ (2005) The proteasome inhibitor MLN-273 blocks exoerythrocytic and erythrocytic development of Plasmodium parasites. Parasitology 131: 37-44.

Mazier D, Beaudoin RL, Mellouk S, Druilhe P, Texier B, et al. (1985) Complete development of hepatic stages of Plasmodium falciparum in vitro. Science 227: 440-442.

Mazier D, Franetich JF, Carraz M, Silvie O, Pino P (2004) Models for studying the effects of herbal antimalarials at different stages of the Plasmodium life cycle. In: Willcox M, Bodeker G, Rasoanaivo P, editors. Traditional medicines for modern times. Boca Raton (Florida): CRC Press. pp. 271-278.

Neerja J, Puri SK (2004) Plasmodium yoelii: Activity of azithromycin in combination with pyrimethamine or sulfadoxine against blood and sporozoite induced infections in Swiss mice. Exp Parasitol 107: 120-124.
Puri SK, Dutta GP (1990) Quinoline esters as potential antimalarial drugs: Effect on relapses of Plasmodium cynomolgi infections in monkeys. Trans Roy Soc Trop Med Hyg 84: 759-760.

Singh N, Puri SK (1998) Causal prophylactic activity of antihistaminic agents against Plasmodium yoelii nigeriensis infection in Swiss mice. Acta Trop 69: 255-260.

Valecha N, Adak T, Bagga AK, Asthana OP, Srivastava JS, et al. (2001) Comparative antirelapse efficacy of CDRI compound 80/53 (Bulaquine) vs primaquine in double blind clinical trial. Curr Sci 80: 561-563.

Walsh DS, Wilairatana P, Tang DB, Heppner DG Jr, Brewer TG, et al. (2004) Randomized trial of 3-dose regimens of tafenoquine (WR238605) versus low-dose primaquine for preventing Plasmodium vivax malaria relapse. Clin Infect Dis 39: 1095-1103.

Zhang Q, Guan J, Sacci JB Jr, Ager AL Jr, Ellis WY, et al. (2005) Unambiguous synthesis and prophylactic antimalarial activities of imidazolidinedione derivatives. J Med Chem 48: 6472-6481.

Basco LK, Ringwald P, Franetich JF, Mazier D. (1999). Assessment of pyronaridine activity in vivo and in vitro against the hepatic stages of malaria in laboratory mice. Trans R Soc Trop Med Hyg 93 (6) : 651-2

Mahmoudi, N., L. Ciceron, J. F. Franetich, K. Farhati, O. Silvie, W. Eling, R. Sauerwein, M. Danis, D. Mazier, and F. Derouin. (2003). In vitro activities of 25 quinolones and fluoroquinolones against liver and blood stage Plasmodium spp. Antimicrob Agents Chemother 47:2636-9.

## Claims

1. Use of at least one compound of the following formula (I): 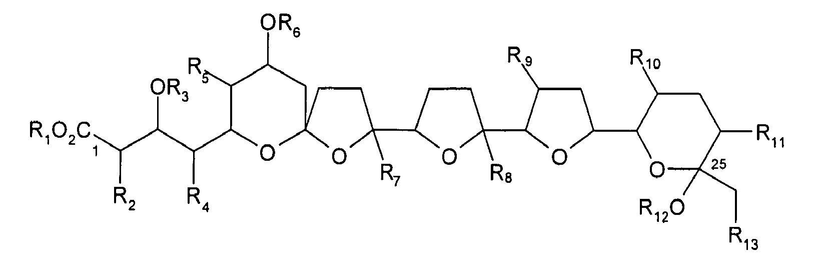 wherein:
- independently from each other, R₁ to R₁₂ represent H or an alkyl group having from 1 to 5 carbon atoms;
- R₁₃ represents a group selected from the list constituted of -OR, -NHR, - OCONHR, -OCOR₁₄, wherein R₁₄ represents a group selected from the list constituted of an alkyl having from 1 to 5 carbon atoms, an aryl having from 5 to 8 carbon atoms, an alkylaryl having from 5 to 13 carbon atoms, an arylalkyl having from 5 to 13 carbon atoms, a haloaryl having from 5 to 8 carbon atoms, an alkylhaloaryl having from 5 to 13 carbon atoms, and a haloarylalkyl having from 5 to 13 carbon atoms; or a pharmacologically acceptable salt thereof, for the manufacture of a medicament intended for preventing or treating infections by a *Plasmodium* parasite in an individual, by inhibiting the pre-erythrocytic development stage of said *Plasmodium* parasite.

2. The use according to claim 1, wherein the compound is of the following formula (II): 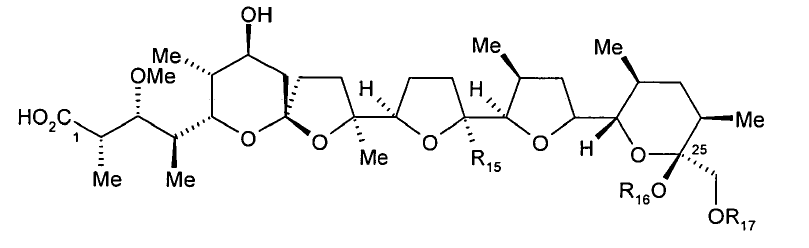 wherein:
R₁₅ represents methyl or ethyl;
R₁₆ represents H or methyl;
R₁₇ represents H or -CONHR₁₈, wherein R₁₈ represents a group selected from the list constituted of phenyl, p-chlorophenyl or p-bromophenyl.

3. The use according to claim 1 or 2, wherein the compound is Monensin A, of the following formula (III):

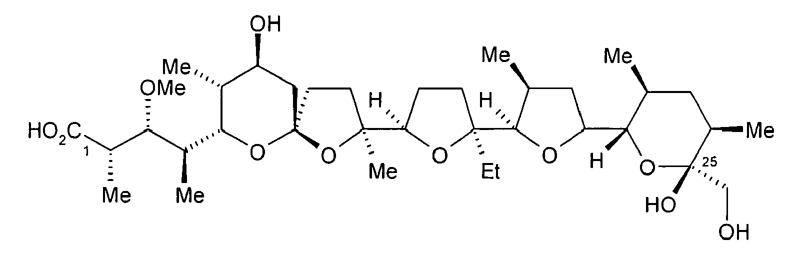

4. The use according to any of claims 1 to 3, wherein the *Plasmodium* parasite is selected from the list constituted of *P*. *falciparum*, *P*. *vivax*, *P*. *ovale*, and *P*. *malariae*.

5. The use according to any of claims 1 to 4, for preventing the infection of the individual by the *Plasmodium* parasite.

6. The use according to any of claims 1 to 5, wherein the individual is a pregnant woman or a child.

7. The use according to any of claims 1 to 6, wherein the medicament is suitable for a dose regimen of from 1 to 15 mg/kg/day of the compound.

8. The use according to any of claims 1 to 7, wherein the medicament comprises a unit dose of from 10 mg to 2 g of the compound.

9. The use according to any of claims 1 to 8, wherein the medicament is suitable for administration by the oral route.

10. The use according to any of claims 1 to 9, wherein the medicament further comprises at least one compound having anti-malarial activity.

11. The use according to claim 10, wherein the compound having anti-malarial activity is selected from the group consisting of Atovaquone, Proguanil, Quinine, Quinidine, Quinine-doxycycline, Artemether, Artemotil, Artesunate, Arteether, Méfloquine, Amodiaquine, Dihydroartémisinine, Pipéraquine, Halofantrine, Atovaquone, Chloroquine, Dapsone, Doxycycline, a Cycline, Lumefanthrine, Proguanil, Pyrimethamine, Pyronaridine, Sulfadoxine, Diamidine, Ferroquine, Fluoroquinolone, Fosmidomycine, Tafenoquine, and Trioxaquine.

12. Products containing:
- at least one compound of formula (I) as defined in any of claims 1 to 3, and
- at least one compound having anti-malarial activity as defined in claims 10 or 11, as a combined preparation for simultaneous, separate or sequential use for the prevention or the treatment of infections by a *Plasmodium* parasite in an individual, by inhibiting the pre-erythrocytic development stage of said *Plasmodium* parasite.
